# EUROPEAN PATENT APPLICATION

(11) **EP 1 985 992 A1**
(43) Date of publication of application: **29.10.2008**
(21) Application number: 07706745.2
(22) Date of filing: 15.01.2007
(51) Int. Cl.: G01N 21/01, G01N 21/59, G01N 35/04, G01N 21/78

(54) **ANALYZER HAVING LIGHT SHIELD**

(30) Priority: 13.01.2006 JP 2006006829
(71) Applicant: Arkray, Inc., Kyoto-shi, Kyoto 601-8045 (JP)
(72) Inventor: UEHATA, Yoshiharu, Kyoto-shi, Kyoto 601-8045 (JP)
(74) Representative: HOFFMANN EITLE
(86) International application number: PCT/JP2007/050411
(87) International publication number: WO 2007/081001

(57) **Abstract**

The invention relates to an analyzing apparatus including an installation portion having an insertion port 43 and provided for installing an analytical tool 20, a light measuring mechanism 22 and 23 irradiating light to the analytical tool 20 and receiving the light making progress from the analytical tool 20 for analyzing a sample in accordance with an optical method, and a light shielding means 63 for limiting an incoming radiation of an external light from the insertion port 43. The light shielding means 63 is structured such as to limit the incoming radiation of the external light via the insertion port 43 after starting the insertion of the analytical tool 20 to the installation portion before the light measurement of the analytical tool 20 is finished in the light measuring mechanism 22 and 23.

## Description

### TECHNICAL FIELD

The present invention relates to an analyzing apparatus structured such as to analyze a sample fed to an analytical tool in accordance with an optical method. More specifically, the present invention relates to an analyzing apparatus provided with a light shielding means for limiting an incoming radiation of an external light into an inner portion of the apparatus.

### BACKGROUND ART

Conventionally, as a method of measuring a blood sugar level, there is a method using an analytical tool. As one example thereof, there is a method of automatically measuring a blood sugar level in a blood sugar level measuring apparatus by installing an analytical tool to a user in a portable blood sugar level measuring apparatus that can be carried around and dotted sampling blood with respect to the analytical tool (for example, refer to Patent Document 1). Further, there is a structure made such as to measure a blood sugar level by combining a cartridge accommodating a plurality of analytical tools with a blood sugar level measuring apparatus and picking up the analyzing tool from the cartridge in the blood sugar level measuring apparatus (for example, refer to Patent Documents 2 and 3).

On the other hand, as a principle for measuring the blood sugar level, for example, there is a principle utilizing an optical method. In the case that the optical method is utilized in the blood sugar level measurement, the analytical tool is structured such as to be provided with a reagent portion indicating a color in correspondence to a concentration of the blood sugar level, however, the blood sugar level measuring apparatus is structured such as to be provided with a light measuring mechanism for measuring a degree of the color of the reagent portion.

However, since the analytical tool used in the portable blood sugar level measuring apparatus is small, it is necessary to set the light measuring mechanism near a portion (an insertion port) where the analytical tool is installed in the case of employing the optical method in the portable blood sugar level measuring apparatus. Accordingly, there is a risk that the light incoming from the insertion port is received in the light measuring mechanism. If such a matter is generated, the matter affects the result of measurement. Further, since the amount of incoming light via the insertion port is affected by a used environment such as a brightness of a used place, a direction of the insertion port in the blood sugar level measuring apparatus and the like, the amount of incoming light is neither uniform in the measurement nor uniform during the measurement. Accordingly, if it is impossible to suitably suppress the light incoming radiation from the insertion port, it is hard to carry out an accurate blood sugar level measurement.

On the other hand, there is a case that a calibration of the light measuring mechanism is carried out until the insertion of the analytical tool to the blood sugar value measuring apparatus is started, and the sample is fed to the analytical tool. This calibration is carried out by outputting the light from a light source portion of the light measuring mechanism, and comprehending the amount of light received in the light receiving portion of the light measuring mechanism. Accordingly, if the amount of the light incoming from the insertion port is changed in the middle of the calibration, the change affects the result of calibration, and is reflected to a measuring precision as a result.

Further, in the case of measuring the light based on the light transmitting through the analytical tool in the light measuring mechanism, there is a case that the light source portion of the light measuring mechanism employs a light source portion provided with a light emitting device for emitting the light, and a light receiving device for monitoring an output from the light emitting device. In this case, if an amount of the light incoming from the insertion port is changed, the output of the light receiving device is erroneously recognized, and the result of measurement is affected as a result.

Patent Document 1: Japanese Unexamined Patent Publication No. 2003-114213
Patent Document 2: Japanese Unexamined Patent Republication No. 01-63272
Patent Document 3: Japanese Unexamined Patent Publication No. 9-184819

### DISCLOSURE OF THE INVENTION

### PROBLEMS TO BE SOLVED BY THE INVENTION

An object of the present invention is to suitably limit an incoming radiation of an external light into an inner portion of an analyzing apparatus via an insertion port and accurately analyze a sample, in the analyzing apparatus structured such as to analyze the sample in accordance with an optical method.

### MEANS FOR SOLVING THE PROBLEMS

In accordance with the present invention, there is provided an analyzing apparatus including an installation portion having an insertion port and provided for installing an analytical tool; a light measuring mechanism irradiating light to the analytical tool and receiving the light making progress from the analytical tool for analyzing a sample in accordance with an optical method; and a light shielding means for limiting an incoming radiation of an external light from the insertion port, wherein the light shielding means is structured such as to limit the incoming radiation of the external light via the insertion port after starting the insertion of the analytical tool to the installation portion before the light measurement of the analytical tool is finished in the light measuring mechanism.

For example, the light shielding means has a contact member for bringing into contact with the analytical tool, in a state in which the analytical tool is installed to the installation portion.

The contact member is structured such as to have a light shielding portion which comes into contact with the analytical tool and is displaceable in a vertical direction. In this case, the contact member may be structured such as to have a leaf spring portion for pressing to the analytical tool via the light shielding portion.

The analyzing apparatus in accordance with the present invention is structured, for example, such as to pick up the analytical tool from the cartridge accommodating a plurality of analytical tools, and analyze the sample by using the analytical tool, and is structured such as to be further provided with an operation body which is made movable so as to reciprocate relatively with respect to a casing, and a movable body capable of reciprocating between a standby position and a pickup position capable of picking up the analytical tool from the cartridge, working with a reciprocating movement of the operation body, and engaging with the analytical tool accommodated in the cartridge so as to pick up the analytical tool from the cartridge, and provided for accommodating at least a part of the analytical tools in an inner portion of the casing. In this case, the light shielding portion is structured such as to be displaced upward by running on the movable body based on the movement of the movable body.

The movable body is provided such as to coming into contact with the light shielding portion at a time when the light shielding portion runs on, and has a guide surface in which a position becomes higher toward an inserting direction of the analytical tool. On the other hand, the light shielding portion is structured, for example, such that a portion coming into contact with the guide surface is formed as an inclined surface or a curved surface.

The contact member may be structured such that the light shielding portion turns. In this case, the light shielding portion is structured, for example, such that a portion brought into contact with the analytical tool is formed as an inclined surface or a curved surface. The light shielding portion may be energized in a direction of pressing the analytical tool. The light shielding portion is constituted, for example, by an elastic member.

The contact member may be structured such as to include a rubber-like elastic body or a foamed body arranged in an inner portion of the insertion port or at a position which is adjacent to the insertion port.

The contact member in this case is structured, for example, such as to have a penetration space through which the analytical tool is inserted. The penetration space is preferably formed such that at least a partial cross sectional area in an orthogonal direction which is orthogonal to the inserting direction of the analytical tool becomes smaller than a cross sectional area in an orthogonal direction in the analytical tool, and is formed, for example, into a taper shape in which the cross sectional area in the orthogonal direction becomes smaller along the inserting direction. The contact member may be structured such as to have a notch to which the analytical tool is inserted.

The light measuring mechanism is structured, for example, such as to include a light emitting device, a first light receiving device utilized for monitoring an output of the light emitted from the light emitting device, and a second light receiving device receiving the light transmitting the analytical tool, in the light emitted from the light emitting device.

The analytical tool is set, for example, to a state in which the analytical tool partly protrudes from the analyzing apparatus after starting the insertion of the analytical tool to the installation portion before the light measurement of the analytical tool is finished in the light measuring mechanism. The analytical tool may be structured such as to suck the sample fed from the portion protruding from the analyzing apparatus into the inner portion thereof.

### BRIEF DESCRIPTION OF THE DRAWINGS

Fig. 1 is a perspective view of an analytical kit for explaining a first embodiment in accordance with the present invention.
Fig. 2 is an overall perspective view of a cartridge in the analytical kit shown in Fig. 1.
Fig. 3A is a cross sectional view taken along a line IIIA-IIIA in Fig. 2A and Fig. 3B is a cross sectional view taken along a line IIIB-IIIB in Fig. 2B.
Fig. 4 is a cross sectional view taken along a line IV-IV in Fig. 2B.
Fig. 5 is an exploded perspective view of an analyzing apparatus shown in Fig. 1.
Fig. 6 is a cross sectional view for explaining a substantial part of the analyzing apparatus shown in Fig. 5.
Fig. 7 is a cross sectional view for explaining the substantial part of the analyzing apparatus shown in Fig. 5.
Fig. 8 is an exploded perspective view of a loading mechanism in the analyzing apparatus shown in Fig. 5.
Fig. 9 is a perspective view of a movable body of the loading mechanism shown in Fig. 8 as seen from a bottom surface side.
Fig. 10 is a perspective view of a second support member of the loading mechanism shown in Fig. 8 as seen from a bottom surface side.
Fig. 11 is a front elevational view showing a movable body and an operation body in the loading mechanism shown in Fig. 8.
Fig. 12 is a cross sectional view showing the movable body and the operation body in the loading mechanism shown in Fig. 8.
Fig. 13 is a cross sectional view showing a substantial part for explaining a motion picking up an analytical tool from the cartridge shown in Figs. 2 and 3.
Fig. 14A is a cross sectional view showing a substantial part of an analyzing apparatus in accordance with a second embodiment of the present invention and Fig. 14B is an overall perspective view showing a pressing member of the analyzing apparatus shown in Fig. 14A.
Fig. 15 is a cross sectional view corresponding to Fig. 14A showing a used state of the analyzing apparatus shown in Fig. 14A.
Fig. 16 is a cross sectional view showing a substantial part of an analyzing apparatus in accordance with a third embodiment of the present invention.
Fig. 17A is a cross sectional view showing a substantial part of an analyzing apparatus in accordance with a fourth embodiment of the present invention, Fig. 17B is an overall perspective view showing a pressing member of the analyzing apparatus shown in Fig. 17A, and Fig. 17C is a cross sectional view corresponding to Fig. 17A showing a used state of the analyzing apparatus shown in Fig. 17A.
Fig. 18A is a cross sectional view showing a substantial part for explaining the other embodiment of the analyzing apparatus in accordance with the present invention and Fig. 18B is an overall perspective view showing a pressing member of the analyzing apparatus shown in Fig. 18A.

### DESCRIPTION OF REFERENCE NUMERALS

- 2: Cartridge
- 20: Analytical tool
- 3: Analyzing apparatus
- 32: Light source apparatus (constructing light measuring mechanism)
- 33: Light receiving portion (constructing light measuring mechanism)
- 4: Casing (of analyzing apparatus)
- 5: Operation body (of analyzing apparatus)
- 6: Movable body (of analyzing apparatus)
- 63: Pressing member (contact member)
- 63A: Light shielding portion (of pressing member)
- 63B: Leaf spring portion (of pressing member)
- 67A: Upper surface side taper surface (guide surface)
- 8, 8',: 8", 9, 9' Analyzing apparatus
- 80: Pressing member (contact member)
- 80', 80": Rotary member
- 90, 90': Elastic member (contact member)
- 91: Through hole (of elastic member)
- 91': Notch (of elastic member)

### BEST MODE FOR CARRYING OUT THE INVENTION

A description will be given below of first to fourth embodiments in accordance with the present invention with reference to the accompanying drawings.

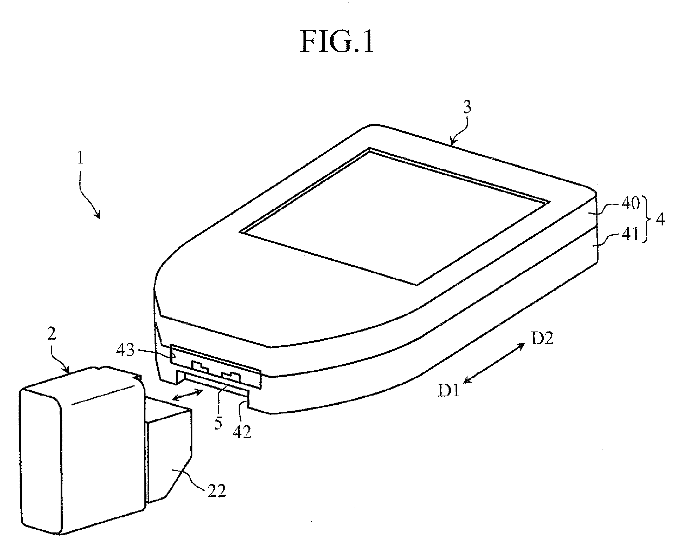

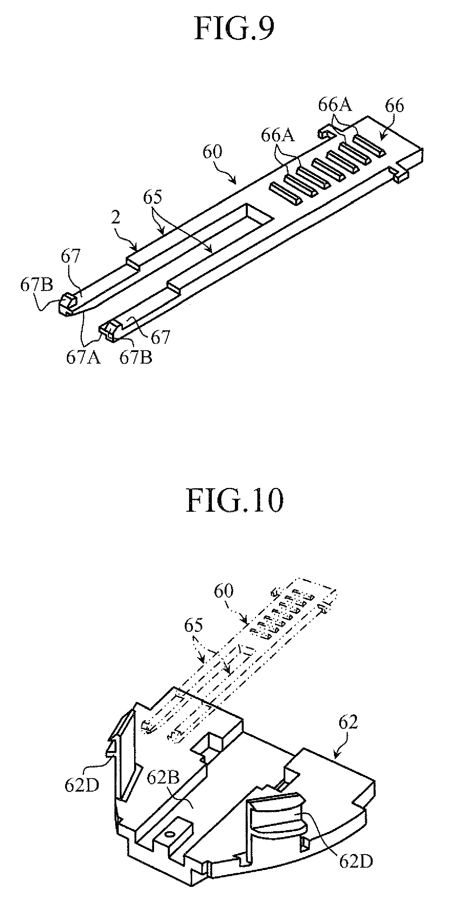

First of all, a description will be given of the first embodiment in accordance with the present invention with reference to Figs. 1 to 13.

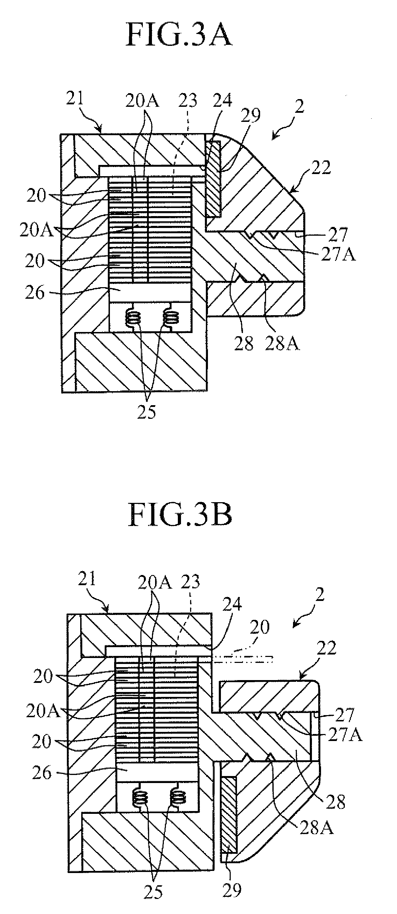

An analytical kit 1 shown in Fig. 1 includes a cartridge 2 (see Fig. 3) accommodating a plurality of analytical tools 20, and an analyzing apparatus 3 for analyzing a sample in accordance with an optical method by using the analytical tool 20. In this analytical kit 1, one analytical tool 20 is picked up from the cartridge 2 by the analyzing apparatus 3 by installing the cartridge 2 to the analyzing apparatus 3, and the analytical tool 20 is installed to the analyzing apparatus 3 and an analysis of the sample is carried out.

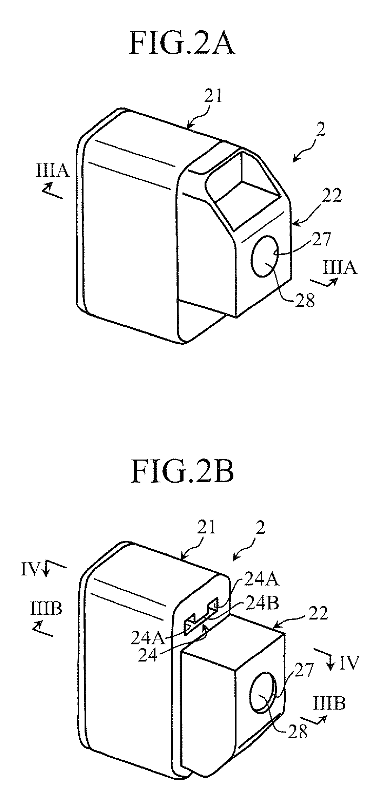

As shown in Figs. 2 and 3, the cartridge 2 is structured such as to accommodate the plurality of analytical tools 20, and has a main body portion 21 and a rotary member 22. The cartridge 2 is structured such that one analytical tool 20 can be picked up by the analyzing apparatus 3.

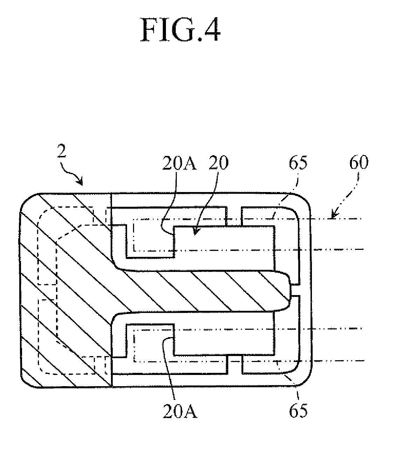

The analytical tool 20 corresponding to an accommodated subject in this cartridge 2 is structured such as to analyze the sample in accordance with the optical method by using a small amount of sample (for example, blood or urine) such as about 0.1 µL to 3 µL. In other words, the analytical tool 20 is structured as a color comparison sensor provided with a reagent portion indicating a color corresponding to a concentration of a detected component in the sample. The analytical tool 20 is structured, for example, such as to feed the sample fed to a suction port to the reagent portion based on a capillary force, and is formed such that at least a part thereof has a light transmitting performance in such a manner as to be capable of measuring in a photometrical manner the reagent portion and a reaction fluid of the sample. As shown in Figs. 3 and 4, each of the analytical tools 20 is formed as a plate-like shaped as a whole, and has a pair of notches 20A. Each of the notches 20A is provided for engaging with an arm portion 65 of a movable body 60 in the analyzing apparatus 3 mentioned below.

As shown in Figs. 2 and 3, the main body portion 21 of the cartridge 2 is provided for accommodating the plurality of analytical tools 20 in a state of laminating in their thickness direction, and has an accommodation space 23 and a pickup port 24.

A support plate 26 coupled to a coil spring 25 is arranged in the accommodation space 23. In other words, the plurality of analytical tools 20 are energized upward by the coil spring 25 in a state in which the analytical tools 20 are laminated on the support plate 26.

The pickup port 24 is utilized at a time of picking up the analytical tool 20 from the accommodation space 23. The pickup port 24 has a pair of wide portions 24A for allowing an insertion of the arm portion 65 of the movable body 60 in the analyzing apparatus 3 mentioned below, and a narrow portion 24B having a thickness corresponding to a thickness of one analytical tool 20.

The rotary member 22 is provided for selecting a state in which the pickup port 24 in the main body portion 21 is exposed and a state (a covered state) in which the pickup port 24 is not exposed, and is rotatably coupled to the main body portion 21. More specifically, the rotary member 22 is coupled to the main body portion 21 by inserting a shaft 28 provided in the main body portion 21 in a through hole 27. In the through hole 27, a spiral convex portion 27A is formed only in one circle although it does not clearly appear on the drawing. On the other hand, the shaft 28 is provided with a spiral groove portion 28A for engaging with the spiral convex portion 27A. Accordingly, the rotary member 22 is relatively rotatable with respect to the main body portion 21 in a state in which the convex portion 28A is engaged with the groove portion 28A, and an angle of rotation is regulated to 180 degree.

The rotary member 22 is further provided with a closely contact member 29. The closely contact member 29 is provided for shielding the pickup port 24 in a state in which the pickup port 24 is covered by the rotary member 22. The closely contact member 29 is constructed by an elastic material such as rubber or the like. In the case that the closely contact member 29 is arranged as mentioned above, it is possible to completely close the pickup port 24 in a state in which the analytical tool 20 is not picked up from the cartridge 2, and it is possible to suppress water content and light from going thereinto via the pickup port 24 so as to suppress a deterioration of the analytic tool 20.

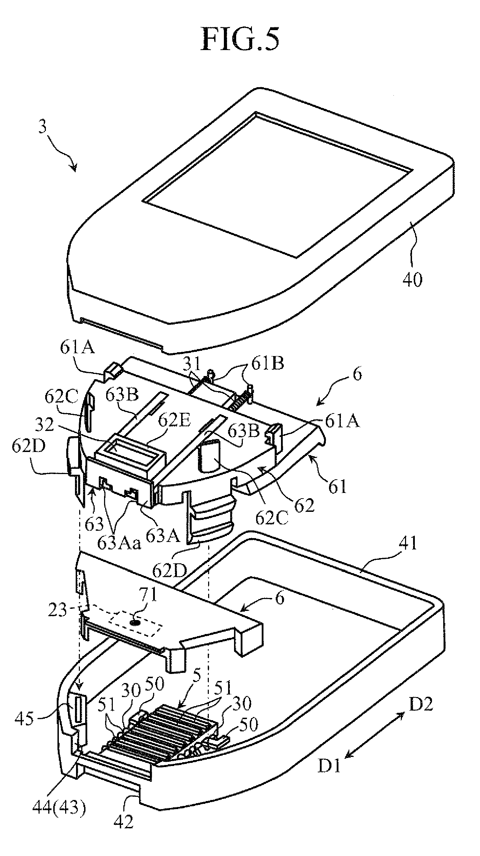

As shown in Figs. 1 and 5, the analyzing apparatus 3 is provided for analyzing the sample in accordance with the optical method by using the analytical tool 20 (see Figs. 3 and 4), and is structured as a portable type. The analyzing apparatus 3 is provided with a casing 4, an operation body 5, a loading mechanism 6 and a light measuring table 7.

The casing 4 is provided for accommodating various elements in addition to the operation body 5 and the loading mechanism 6 as well as defining an outer appearance shape of the analyzing apparatus 3. The casing 4 is formed hollow by combining the first and second members 40 and 41, and has an opening 42 and an insertion port 43.

The opening 42 is provided for making the operation body 5 be exposed to an outer portion of the casing 4, and allowing a movement of the operation body 5, and is provided in the second member 41. The insertion port 43 is utilized at a time of installing the analytical tool 20 (see Figs. 2 and 3) to the analyzing apparatus 3. The insertion port 43 is defined by a notch 44 provided in the second member 41.

As shown in Figs. 5 to 7, the operation body 5 is a portion which is operated for moving the movable body 60 in the loading mechanism 6 mentioned below, and is structured such as to freely reciprocate in directions D1 and D2 with respect to the casing 4 in a state in which the operation body 5 is partly exposed from the opening 42. The operation body 5 has a pair of hook portions 50 and a plurality of tooth portions 51.

Each of the hook portions 50 is a portion with which one end portion of the coil spring 30 is engaged. The other end portion of the coil spring 30 is fixed near the opening 42 of the second member 41 in the casing 4, although it is not clearly expressed in the drawing. The operation body 5 is energized in the direction D1 by the coil spring 30 in a standby state. Accordingly, the operation body 5 is structured such as to move in the direction D2 at a time when a load directed to the direction D2 is applied, and return to the standby position at a time when the load in the direction D2 is cancelled.

A plurality of tooth portions 51 are a portion which is utilized for transmitting a power to the movable body 60, and are coupled to a rack portion 66 of the movable body 60 mentioned below via a gear 64 in the loading mechanism 6.

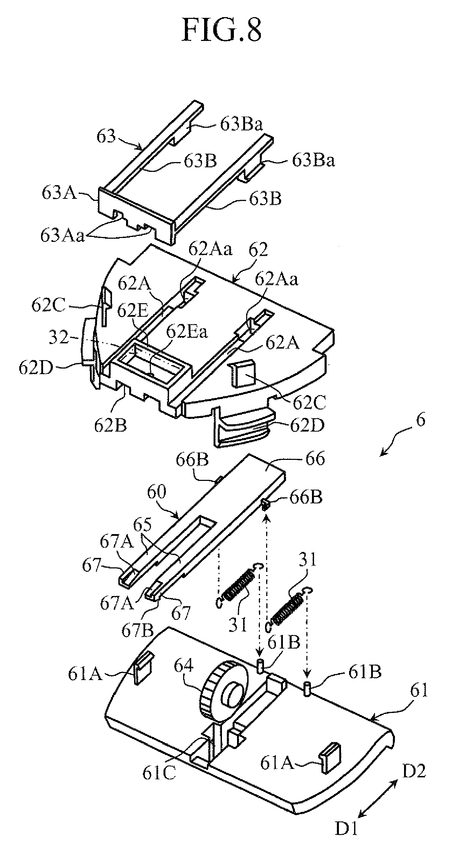

As shown in Figs. 5 and 8, the loading mechanism 6 is provided for picking up the analytical tool 20 (see Figs. 2 to 4) from the cartridge 2, and has a movable body 60, a first support member 61, a second support member 62, a pressing member 63 and a gear 64.

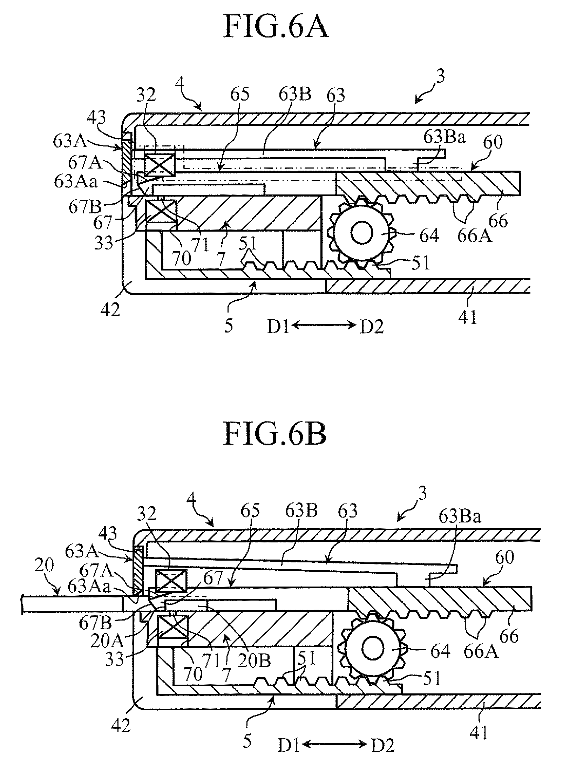

As shown in Figs. 6 to 9, the movable body 60 is moved in the directions D1 and D2 working with a motion of the operation body 5, and has a pair of arm portions 65 and a rack portion 66.

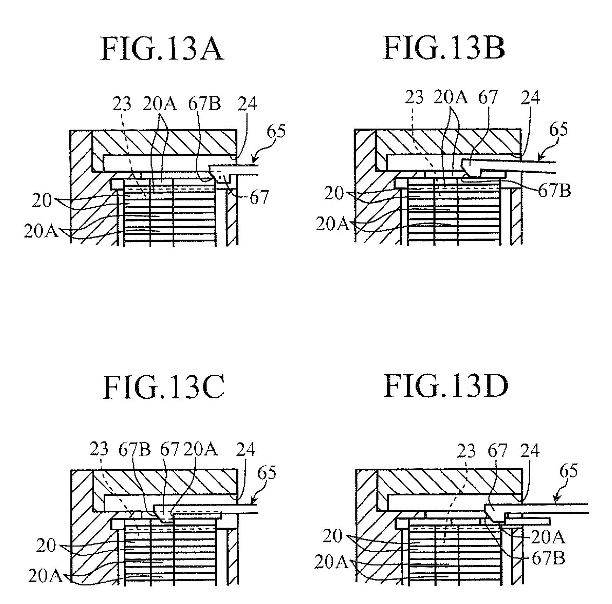

A pair of arm portions 65 are provided for picking up the analytical tool 20 from the cartridge 2. These arm portions 65 are reciprocated between a position at which the arm portions 65 protrude from the casing 4 and a position at which the arm portions 65 are accommodated in an inner portion of the casing 4, and can be inserted to the accommodation space 23 in the cartridge 2 by being protruded from the casing 4 (see Fig. 13).

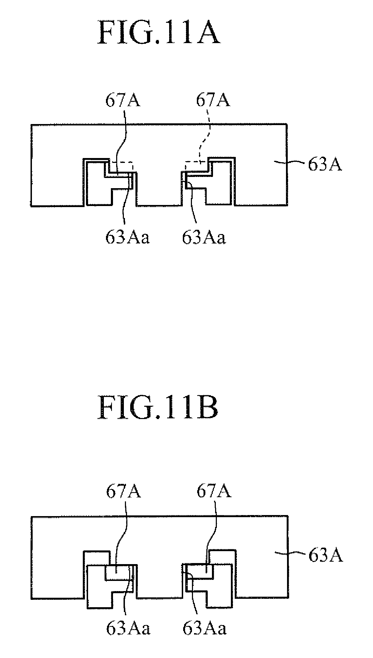

Each of the arm portions 65 has a hook portion 67 for engaging with the notch 20A (see Figs. 4 and 13) of the analytical tool 20. The hook portion 67 has an upper face side taper surface 67A and a lower face side taper surface 67B. The upper face side taper surface 67A is a portion which comes into contact with the light shielding portion 63A of the pressing member 63 mentioned below (see Fig. 11), and plays a role for moving the light shielding portion 63A up and down, at a time of relatively moving a pair of arm portions 65 (the movable body 60) in the directions D1 and D2 with respect to the light shielding portion 63A (the pressing member 63).
On the other hand, the lower face side taper surface 67B is provided for easily running on an upper surface of the analytical tool 20 at a time of picking up the analytical tool 20 from the cartridge 2 (see Fig. 13).

The rack portion 66 is utilized for inputting a load for moving the movable body 60, and has a plurality of tooth portions 66A and locking pieces 66B. A plurality of tooth portions 66A are coupled to a plurality of tooth portions 51 of the operation body 5 via the gear 64. The locking piece 66B is provided for locking the coil spring 31.

As shown in Figs. 5 and 8, the first support member 61 defines a moving path of the movable body 60 together with the second support member 62, and retains the gear 64. The first support member 61 has a pair of hook portions 61A, a pair of pins 61B and a holder portion 61C.

A pair of hook portions 61A are provided for engaging with the second support member 62, and are protruded upward. In a state in which each of the hooks 61A is engaged with the second support member 62, the first and second members 61 and 62 are integrated with each other. A pair of pins 61B are provided for locking one end portion of the coil spring 31. The holder portion 61C is provided for rotatably retaining the gear 64. The holder portion 61C is structured such as to retain the gear 64 in such a manner that a part of the gear 64 protrudes to an upper side and a lower side of the first support member 61. In other words, the gear 64 is retained in the first support member 62 in a state in which the gear 64 can be engaged with the plurality of tooth portions 51 and 66A of the operation body 5 and the movable body 60.

As shown in Figs. 5, 8 and 10, the second support member 62 defines a moving path of the movable body 60 together with the first support member 61, and is provided for retaining the pressing member 63 and fixing the loading mechanism 6 to the casing 4. The second support member 62 has a pair of retaining grooves 62A, a guide concave portion 62B, a pair of first and second hook portions 62C and 62D, and a holder portion 62E.

A pair of retaining grooves 62A are provided for accommodating a leaf spring portion 63B of the pressing member 63 mentioned below, and extend in the directions D1 and D2. A through hole 62Aa is provided at a position close to an end portion in the direction D2 side, in each of the retaining grooves 62A. The through hole 62Aa is provided for inserting a hook portion 63Ba of the pressing member 63 mentioned below.

The guide concave portion 62B accommodates the arm portion 65 in the movable body 60, and is provided for defining a moving path of the arm portion 65 (the movable body 60).

A pair of first and second hook portions 62C and 62D are provided for fixing the second support member 62 and therefore the loading mechanism 6 to the casing 4. Each of the first hook portions 62C is provided for engaging with the first member 40 in the casing 4, and protrudes upward. On the other hand, the second hook portion 62D is provided for engaging with the engagement portion 45 of the second member 41 in the casing 4, and protrudes toward a lower side.

The holder portion 62E is provided for retaining the light source apparatus 32. The light source apparatus 32 constructs a light measuring mechanism for measuring in a photometrical manner the analytical tool 20, and is provided at a position facing to a reagent portion (not shown) of the analytical tool 20 in a state in which the analytical tool 20 is installed to the analyzing apparatus 3. The light source apparatus 32 includes a light emitting device and a light receiving device although they are omitted on the drawing, and is structured such as to directly receive a part of the light emitted from the light emitting device on the light receiving device, and be capable of monitoring an output of the light emitting device. The holder 62E is provided with a through hole 62Ea. The through hole 62Ea is provided just below a light emitting surface (not shown) in the light emitting element, and plays a role for guiding the light emitted from the light source apparatus 32 (the light emitting device) to a lower side of the second support member 62. In other words, it is structured such that only the light transmitting the through hole 62Ea is irradiated to the reagent portion (not shown) of the analytical tool 20.

As shown in Figs. 5 to 8 and Figs. 11 and 12, the pressing member 63 is provided for limiting the incoming radiation of the external light from the insertion port 43 of the casing 4. The pressing member 63 has the light shielding portion 63A and a pair of leaf spring portions 63B.

The light shielding portion 63A comes into contact with the movable body 60 at a time of moving the movable body 60 in the directions D 1 and D2. In other words, the light shielding portion 63A is structured such as to be displaced in a vertical direction based on the movement of the arm portion 65 as well as closing the insertion port 43 in a standby state. A notch 63Aa is provided in the light shielding portion 63A. The notch 63Aa is provided for allowing the movement of the arm portion 65 in the movable body 60, and a portion coming into contact with the upper face side taper surface 67A in the hook portion 67 of the arm portion 65 is formed as an inclined taper surface. The light shielding portion 63A can easily run on the upper surface of the arm portion 65 at a time when the arm portion 65 protrudes from the casing 4, based on the taper surface.

A pair of leaf spring portions 63B are provided for applying a downward pressing force to the light shielding portion 63A as well as slidably supporting the light shielding portion 63A. Each of the leaf spring portions 63B has a hook portion 63Ba, and is fixed to the second support member 62 in the hook portion 63Ba. Each of the leaf spring portions 63B has a spring characteristic, and is arranged in such a manner as to deflect at a time when an upward force is applied thereto via the light shielding portion 63A. Accordingly, the pressing member 63 is structured such as to apply a downward snapping force to the light shielding portion 63 at a time when the light shielding portion 63A is displaced upward.

As shown in Figs. 6 to 8, the gear 64 is engaged with each of the plurality of tooth portions 51 and 66A of the operation body 5 and the movable body 60 as mentioned above, and is rotatably retained in the first support member 61. In other words, the gear 64 is rotated by moving the operation body 5, plays a role in transmitting a rotating force at that time to the movable body 60, and is provided for moving the movable body 60 in an opposite direction to the moving direction of the operation body 5. More specifically, in the case that the load directed to the direction D2 is applied to the operation body 5, the movable body 60 moves in the direction D1, the arm portion 65 largely protrudes from the casing 4, and there is achieved a state in which the arm portion 65 can be inserted to the main body portion 21 of the cartridge 2. On the contrary, in the case that the load applied to the operation body 5 is canceled and the operation body 5 is moved in the direction D1, the movable body 60 is moved in the direction D2, and the arm portion 65 is set to a state of being accommodated in the inner portion of the casing 4.

As shown in Figs. 5 and 6, a light measuring table 7 is structured such as to be supported to the second member 41 of the casing 4, and corresponds to a portion to which the analytical tool 20 picked up by the loading mechanism 6 is mounted. The light measuring table 7 is provided with a retaining portion 70 for retaining the light receiving portion 33. The retaining portion 70 is provided with a through hole 71 at a position just below a through hole 62Ea of a holder 62E in the second support member 62.

The light receiving portion 33 is structured such as to construct a light measuring mechanism together with the light source apparatus 32, and receive the light transmitting through the analytical tool 20 in the light emitted from the light source apparatus 32 and irradiated to the analytical tool 20. In other words, the amount of light received in the light receiving portion 33 correlates with a light absorbing characteristic (a level of color) in the reagent portion (not shown) of the analytical tool 20, and it is possible to compute a concentration of a detected component in the sample based on the previous amount of light received.

Next, a description will be given of an analyzing method of the sample by using the analytical kit 1, and operation of the cartridge 2 and the analyzing apparatus 3.

In the case of analyzing the sample by using the analytical kit 1, first of all, the analytical tool 20 accommodated in the cartridge 2 is fed to the analyzing apparatus 3. The analytical tool 20 is fed to the analyzing apparatus 3 by detaching the cartridge 2 from the analyzing apparatus 3 after installing the cartridge 2 to the analyzing apparatus 3.

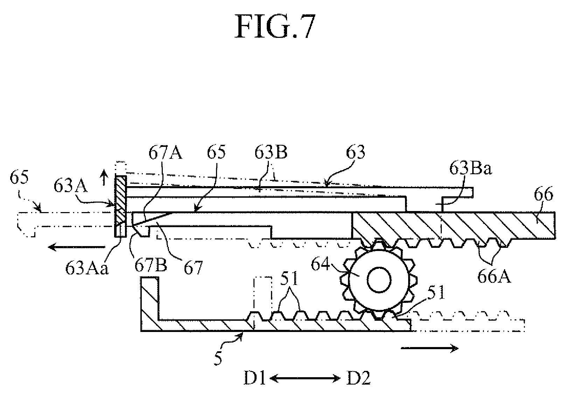

The cartridge 2 is installed to the analyzing apparatus 3 in a state in which the pickup port 24 is open by turning the rotating member 22 in the cartridge 2 at 180 degree in accordance with a manual operation of a user as shown in Figs. 2 and 3. If the cartridge 2 is installed to the analyzing apparatus 3 in this state, the force directed in the direction D2 is applied to the operation body 5 by the rotating member 22 of the cartridge 2. Accordingly, as shown in Fig. 7, the movable body 60 is moved in an opposite direction (the direction D1) to the operation body 5.

As shown in Figs. 6, 7, 11 and 12, in the process in which the movable body 60 moves, the arm portion 65 moves while coming into contact with an inner surface (a taper surface) of the notch 63Aa of the light shielding portion 63A in the pressing member 63, in the upper face side taper surface 67A of the hook portion 67. Accordingly, the light shielding portion 63A is displace upward, and the downward snapping force is applied to the arm portion 65 due to the deflection of the leaf spring portion 63B.
As a result, the arm portion 65 moves in the direction D1 in a state in which the arm portion 65 is closely attached to the notch 63Aa of the light shielding portion 63A.

On the other hand, in the case that the movable body 60 is moved in the direction D1, the arm portion 65 protrudes from the casing 4 via the insertion port 43, and the arm portion 65 is inserted to the inner portion of the cartridge 2 from the pickup port 24 of the cartridge 2, as shown in Fig. 13A. Further, since the arm portion 65 is structured such that the hook portion 67 has the lower face side taper surface 67B, the arm portion 65 runs on the upper surface of the analytical tool 20 accommodated in the highest portion in the cartridge 2, as shown in Fig. 13B, and the hook portion 63 thereafter gets down the notch 20A of the analytical tool 20 so as to be engaged with the notch 20A, as shown in Fig. 13C.

On the contrary, in the case that the cartridge 2 is detached from the analyzing apparatus 3 (in the case that the cartridge 2 is moved relatively in the direction D1 with respect to the analyzing apparatus 3), the load directed to the direction D2 applied to the operation body 5 is canceled, and the operation body 5 is moved in the direction D1 so as to be returned to the original position by the snapping force of the coil spring 31 (see Figs. 5 and 8), as is known from Figs. 6 and 7 and the like.

The movable body 60 moves in the direction D2 so as to be returned to the original position while working with the movement in the direction D1 of the operation body 5. At this time, since the hook portion 63 in the movable body 60 is engaged with the notch 20A of the analytical tool 20, the analytical tool 20 is moved in the direction D2 together with the movable body 60 and is detached from the cartridge 2, as shown in Fig. 13D, and the end portion 20B of the analytical tool 20 is accommodated in the inner portion of the casing 4, as shown in Fig. 6B. More specifically, the analytical tool 20 is structured such that the end portion 20B is mounted on the light measuring table 7 in such a manner that the reagent portion (not shown) is positioned between the light source apparatus 32 and the light receiving portion 33 in the light measuring mechanism, and a part of the analytical tool 20 protrudes from the analyzing apparatus 3. In this state, it is possible to measure light of the reagent portion (not shown) in the analytical tool 20 by the light measuring mechanisms 32 and 33.

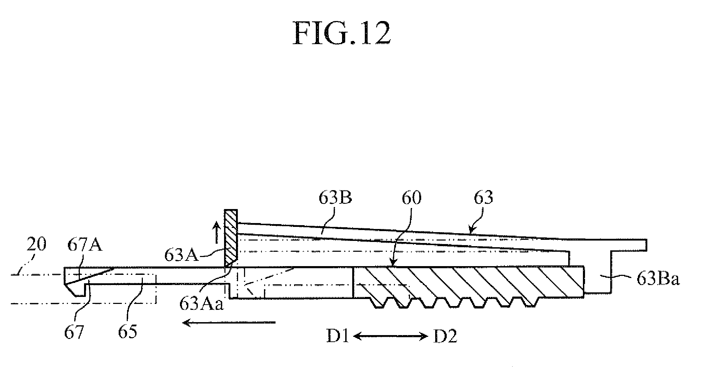

In the process of moving the analytical tool 20 in the direction D2 together with the movable body 60, a state in which the arm portion 65 comes into contact with the light shielding portion 63A is maintained, at first, as is known from Fig. 12. Further, in the case that the arm portion 65 is moved to the inner portion than the insertion port 43, the contact state between the light shielding portion 63A and the arm portion 65 is canceled. At this time, the light shielding portion 63A is displaced downward by the snapping force from the leaf spring portion 63B, however, since a part of the analytical tool 20 exists in the D1 side of the arm portion 65, the light shielding portion 63A comes into contact with the upper surface of the analytical tool 20 as shown in Fig. 6B. In this contact state, since the light shielding portion 63A is kept in the state of being displaced upward in comparison with the original position, the state in which the leaf spring portion 62B is deflected is maintained. As a result, the light shielding portion 63A is closely attached to the analytical tool 20 in a state in which the light shielding portion 63A applies the pressing force to the upper surface of the analytical tool 20, and in the case of measuring the light of the analytical tool 20, the insertion port 43 is suitably closed by the light shielding portion 63A.

Further, in the case that the installation of the analytical tool 20 to the analyzing apparatus 3 is finished, the light is irradiated to the reagent portion (not shown) of the analytical tool 20 from the light source apparatus 32 in the light measuring mechanism, and the light transmitting the analytical tool 20 (the reagent portion) is received by the light receiving portion 33 at that time. In the analyzing apparatus 3, the level of the color of the reagent portion is comprehended based on the result of light receiving in the light receiving portion 33, and the concentration of the detected component in the sample is computed.

In the case that the analysis of the sample is finished, it is necessary to dispose the analytical tool 20, however, the analytical tool 20 can be disposed by moving the operation body 5 in the direction D2 by the user. In other words, in the case of moving the operation body 5 in the direction D2, the movable body 60 is moved in the direction D1, and the analytical tool 20 is also moved in the direction D1. Further, in the case that the movable body 60 is moved to the pickup position, a whole of the analytical tool 20 comes to a state of jumping out of the casing 4, and comes down due to its own weight, and the analytical tool 20 can be disposed from the analyzing apparatus 3.

In the analytical kit 1, the analytical tool 20 is installed to the analyzing apparatus 3 by detaching the cartridge 2 from the analyzing apparatus 3 after installing the cartridge 2 to the analyzing apparatus 3. Further, in the installing process of the analytical tool 20, the light shielding portion 63A of the pressing member 63 is closely attached to the arm portion 65, and is then closely attached to the analytical tool 20. Accordingly, since the insertion port 43 of the analyzing apparatus 3 is maintained in the state of being suitably closed by the arm portion 65 or the analytical tool 20 and the light shielding portion 63A, it is possible to suitably suppress the external light from incoming from the insertion port 43 in the process of inserting the analytical tool 20 via the insertion port 43 of the analyzing apparatus 3. As a result, even in the case that the light measuring mechanisms 32 and 33 are calibrated during the term when the sample is fed to the analytical tool, it is possible to inhibit the external light from being received in the light receiving portion 33 at a time of this calibration. Accordingly, it is possible to suitably calibrate the light measuring mechanisms 22 and 23, and it is possible to suppress a disadvantage caused by the incoming radiation of the external light at a time of calibrating, that is, a deterioration of a measuring precision.

Further, in the case of measuring the analytical tool 20 in the photometrical manner, since the light shielding portion 63A is closely attached to the analytical tool 20, it is possible to suitably limit the incoming radiation of the external light from the insertion port 43 at a time of measuring the light. Accordingly, it is possible to suppress the influence of the external light at a time of measuring the light, and it is possible to carry out a more suitable sample analysis.

Further, the light shielding portion 63A of the pressing member 63 closes the insertion port 43 in the standby state, and even in the process of installing the analytical tool 20 to the analyzing apparatus 3 and in the case of measuring the analytical tool 20 in the photometrical manner, the insertion port 43 is suitably closed. Accordingly, even in the case of monitoring the output of the light emitting device of the light source apparatus 32 in the light measuring mechanisms 32 and 33, it is possible to monitor the output of the light emitting device in the light source apparatus 32 while suppressing the influence of the external light, whichever case the timing of the output monitor is the standby state, the inserting process of the analytical tool 20 and under measurement of the analytical tool 20 in the photometrical manner. As a result, since it is possible to suitably comprehend the output of the light emitting device of the light source apparatus 32, it is possible to suppress the deterioration of the measuring precision based on an erroneous recognition of the output of the light emitting device.

Accordingly, in the analyzing apparatus 3, it is possible to inhibit the measuring precision from being deteriorated by the external light, and inhibit a dispersion from being generated in the result of measurement.

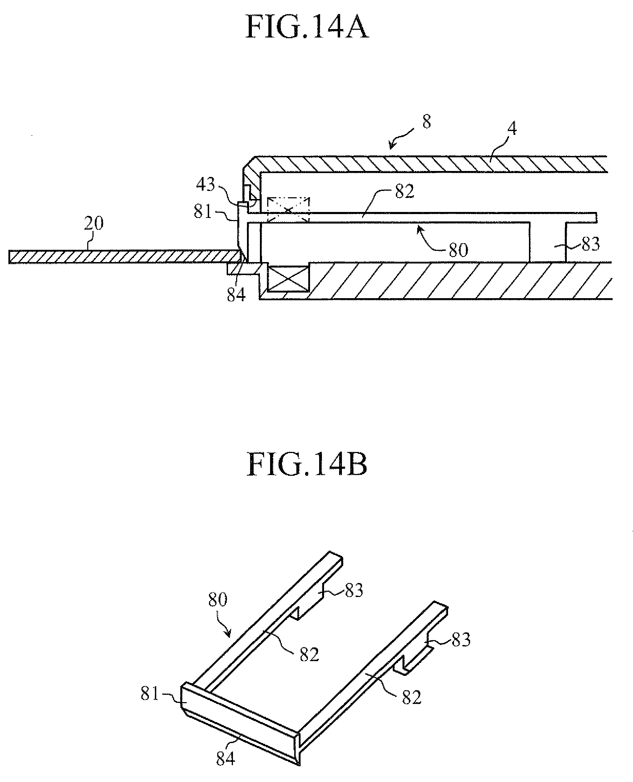

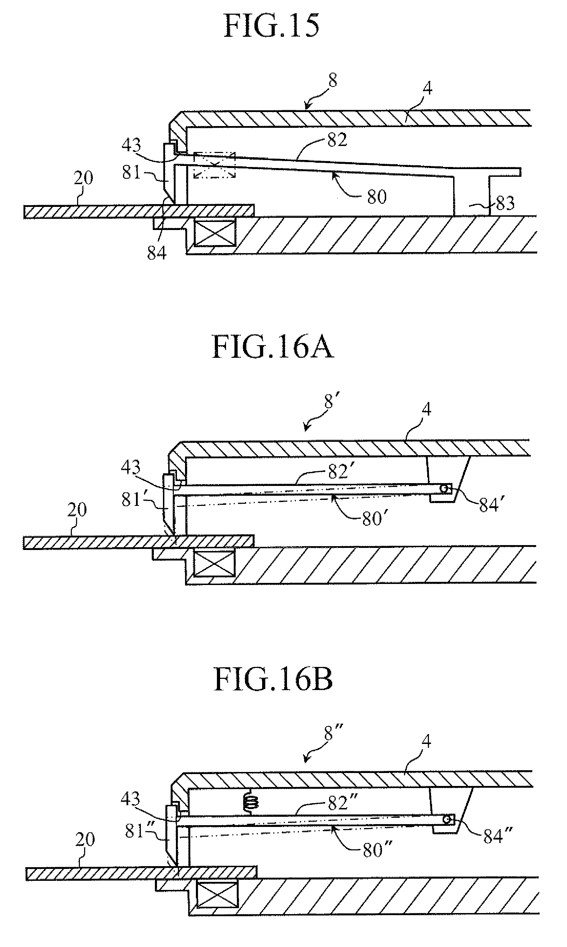

Next, a description will be given of an analyzing apparatus in accordance with a second embodiment of the present invention with reference to Figs. 14 and 15. In this case, in Figs. 14 and 15, the same reference numerals are attached to the same members and elements as those of the first embodiment in accordance with the present invention, and an overlapping description will be omitted.

An analyzing apparatus 8 shown in Fig. 14A is structured such as to install the analytical tool 20 in accordance with a manual operation of a user without using any cartridge 2 (see Figs. 2 and 3).

The analyzing apparatus 8 is provided with a pressing member 80 in the same manner as the analyzing apparatus 3 (see Fig. 6 and the like) described previously. The pressing member 80 has a light shielding portion 81 and a pair of leaf springs 82 as shown in Fig. 14B, and is directly or indirectly fixed to the casing 4, in a hook portion 83 of each of the leaf spring portions 82. Accordingly, in the pressing member 80, the light shielding portion 81 can be slidably moved in the vertical direction based on a spring characteristic of the leaf spring portion 82.

On the other hand, the light shielding portion 81 is formed as a rectangular plate shape in which a lower end edge is formed as a linear shape, and closes the insertion port 43 in its standby state. In other words, since the analyzing apparatus 8 is not structured such as to move the arm portion 65 such as the previous analyzing apparatus 3, the notch 63Aa (see Fig. 11) for allowing the movement of the arm portion 65 is not provided in the light shielding portion 82. Further, a lower end portion of the light shielding portion 81 has a taper surface 84 in which a height position becomes higher toward the direction D1. The taper surface 84 corresponds to a portion with which the analytical tool 20 interferes at a time of inserting the analytical tool 20, and is provided for facilitating the insertion of the analytical tool 20.

As shown in Fig. 15, in the analyzing apparatus 3, the analytical tool 20 is inserted from the insertion port 43 in such a manner as to make the end portion of the analytical tool 20 interfere with the taper surface 84 of the light shielding portion 81. At this time, the light shielding portion 81 does not obstruct the insertion of the analytical tool 20 by the taper surface 84, and the end portion of the analytical tool 20 moves along the taper surface 84. Accordingly, the light shielding portion 81 is displaced upward, however, the leaf spring portion 82 is deflected based on the upward displacement of the light shielding portion 81. As a result, the light shielding portion 81 presses the analytical tool 20 downward in the inserting process of the analytical tool 20, and the state in which the light shielding portion 81 is closely attached to the analytical tool 20 is maintained. Of course, even in the case that the installation of the analytical tool 20 to the analyzing apparatus 3 is finished and the measurement in the photometrical manner of the analytical tool 20 is carried out, the state in which the analytical tool 20 is pressed by the light shielding portion 81 is maintained.

As mentioned above, in the analyzing apparatus 8, the analytical tool 20 is pressed by the light shielding portion 81 continuously in the inserting process of the analytical tool 20 and at a time of measuring the light. Accordingly, in the state in which the insertion port 43 is suitably closed by the light shielding portion 81, the analytical tool 20 is inserted, and the measurement in the photometrical manner of the analytical tool 20 is carried out. As a result, in the same manner as the analyzing apparatus 3 (see Figs. 5 to 11) described previously, it is possible to suppress the influence of the external light at a time of the calibration, at a time of the measurement in the photometrical manner, and at a time of monitoring the output of the light emitting device (not shown) in the light emitting apparatus 22. Accordingly, in the analyzing apparatus 8, it is possible to inhibit the measuring precision from being deteriorated by the external light, and inhibit the dispersion from being generated in the result of measurement.

Next, a description will be given of a third embodiment in accordance with the present invention with reference to Figs. 16A and 16B. In these drawings, the same reference numerals are attached to the same member and elements as those of the first embodiment in accordance with the present invention, and an overlapping description will be omitted.

An analyzing apparatus 8' shown in Fig. 16A is different from the analyzing apparatus 8 (see Figs. 14 and 15) described previously in a point that a rotary member 80' serving as the light shielding means is rotatably supported to the casing 4. The pressing member 80' is structured such that a light shielding portion 81' turns around a shaft portion 84', whereby the light shielding portion 81' displaces in the vertical direction.

On the other hand, an analyzing apparatus 8" shown in Fig. 16B is structured such that the rotary member 80' is rotatably supported to the casing 4, and a beam portion 82' is pressed downward by a coil spring 85'. In other words, a light shielding portion 81" is structured such that a downward force is applied based on a snapping force of the coil spring 85'.

In this case, in the analyzing apparatuses 8' and 8", it is not necessary that the rotary members 80' and 80" are supported to the casing 4, but the rotary members 80' and 80" may be supported to the other element than the casing 4. Further, in the analyzing apparatus 8", the downward force may be applied to the rotary member 80" by the other member than the coil spring 85'.

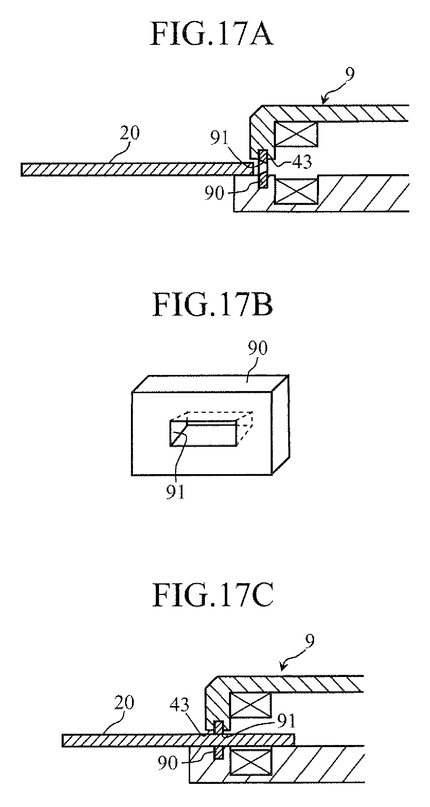

Next, a description will be given of an analyzing apparatus in accordance with a fourth embodiment of the present invention with reference to Fig. 17.

The analyzing apparatus 9 shown in Fig. 17A is the same as the analyzing apparatuses 8, 8' and 8" (see Figs. 14 to 16) described previously in a point that the analyzing apparatus 9 is structured such as to install the analytical tool 20 in accordance with the manual operation of the user, without using any cartridge 2 (see Figs. 2 and 3), however, the structure of the light shielding means is different from the analyzing apparatuses 8, 8' and 8".

As shown in Fig. 17B, in the analyzing apparatus 9, an elastic body 90 is employed as the light shielding means. The elastic body 90 is constructed by a rubber-like elastic body or a foamed body, and is formed as a rectangular ring shape having a through hole 91. The elastic body 90 is arranged in the insertion port 43 as shown in Figs. 17A and 17B, and is substantially structured such as to insert the analytical tool 20 via the through hole 91.

The through hole 91 passes through in the directions D1 and D2, and is structured such that a cross sectional area becomes smaller toward an inner portion of the casing 4. A minimum cross sectional area of the through hole 91 is made smaller than the cross sectional area of the analytical tool 20. Accordingly, in the case of inserting the analytical tool 20 via the through hole 91, the through hole 91 is expanded, and a snapping force of the pressing member 90 is applied to a whole of the periphery of the analytical tool 20. Therefore, since the insertion port 43 is set to the state of being suitably closed, at a time of the calibration, at a time of the measurement in the photometrical manner, and at a time of monitoring the output of the light emitting device (not shown) in the light emitting apparatus 22, it is possible to suppress the influence of the external light. Accordingly, in the analyzing apparatus 9, it is possible to inhibit the measuring precision from being deteriorated by the external light, and inhibit the dispersion from being generated in the result of measurement.

Of course, the present invention is not limited to the analyzing apparatuses described in the first to third embodiments, but can be variously designed and modified. For example, the light measuring mechanism in each of the embodiments is structured as the transmitting type, however, the light measuring mechanism may be structured as a reflecting type.

Further, in the first embodiment in accordance with the present invention, the mechanism for picking up the analytical tool 20 from the cartridge 2 can be variously changed. For example, the hook portion 67 in the arm portion 65 may be formed as a curved surface in place of the inclined surface in the lower face side taper surface 67B.

The present invention can be further applied to an analyzing apparatus used by accommodating a plurality of analytical tools in an inner portion and setting the analytical tools in the inner portion of the apparatus.

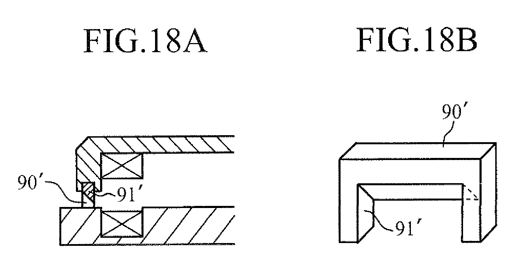

In the analyzing apparatus 9 in accordance with the fourth embodiment of the present invention, the elastic body 90 is formed as the rectangular ring shape, however, the pressing member 90 may be structured such as to press at least a part of the analytical tools 20. Accordingly, the through hole 91 may be structured such as to have a uniform cross section, and may be arranged at a position which is adjacent to the insertion port 43 in place of the inner portion of the insertion port 43. Further, it is not necessary that the elastic body presses the whole of the periphery of the analytical tool 20, for example, an elastic body 90' may be formed as a shape having a partly segmented notch 91', or may be formed as a rod-like shape, or may be constituted by a plurality of parts, as shown in Figs. 18A and 18B.

## Claims

1. An analyzing apparatus having light shielding means comprising:
an installation portion having an insertion port and provided for installing an analytical tool;
a light measuring mechanism irradiating light to the analytical tool and receiving the light making progress from the analytical tool for analyzing a sample in accordance with an optical method; and
the light shielding means for limiting an incoming radiation of an external light from the insertion port,
wherein the light shielding means is structured such as to limit the incoming radiation of the external light via the insertion port after starting the insertion of the analytical tool to the installation portion before the light measurement of the analytical tool is finished in the light measuring mechanism.

2. The analyzing apparatus having the light shielding means as claimed in claim 1, wherein the light shielding means has a contact member for bringing into contact with the analytical tool, in a state in which the analytical tool is installed to the installation portion.

3. The analyzing apparatus having the light shielding means as claimed in claim 2, wherein the contact member has a light shielding portion which comes into contact with the analytical tool and is displaceable in a vertical direction.

4. The analyzing apparatus having the light shielding means as claimed in claim 2, wherein the contact member has a leaf spring portion for applying a pressing force to the analytical tool via the light shielding portion.

5. The analyzing apparatus having the light shielding means as claimed in claim 3, wherein the analyzing apparatus is structured such as to pick up the analytical tool from the cartridge accommodating a plurality of analytical tools, and analyze the sample by using the analytical tool, and wherein the analyzing apparatus further comprises:
an operation body which is made movable so as to reciprocate relatively with respect to a casing; and
a movable body capable of reciprocating between a standby position and a pickup position capable of picking up the analytical tool from the cartridge, working with a reciprocating movement of the operation body, and engaging with the analytical tool accommodated in the cartridge so as to pick up the analytical tool from the cartridge, and provided for accommodating at least a part of the analytical tools in an inner portion of the casing,
wherein the light shielding portion is structured such as to be displaced upward by running on the movable body based on the movement of the movable body.

6. The analyzing apparatus having the light shielding means as claimed in claim 5, wherein the movable body is provided such as to coming into contact with the light shielding portion at a time when the light shielding portion runs on, and has a guide surface in which a position becomes higher toward an inserting direction of the analytical tool.

7. The analyzing apparatus having the light shielding means as claimed in claim 5, wherein the light shielding portion is structured such that a portion coming into contact with the guide surface is formed as an inclined surface or a curved surface.

8. The analyzing apparatus having the light shielding means as claimed in claim 3, wherein the contact member is structured such that the light shielding portion turns.

9. The analyzing apparatus having the light shielding means as claimed in claim 8, wherein the light shielding portion is energized in a direction of pressing the analytical tool.

10. The analyzing apparatus having the light shielding means as claimed in claim 9, wherein the light shielding portion is energized in the direction of pressing the analytical tool by an elastic member.

11. The analyzing apparatus having the light shielding means as claimed in claim 2, wherein the contact member includes a rubber-like elastic body or a foamed body arranged in an inner portion of the insertion port or at a position which is adjacent to the insertion port.

12. The analyzing apparatus having the light shielding means as claimed in claim 11, wherein the contact member has a penetration space through which the analytical tool is inserted.

13. The analyzing apparatus having the light shielding means as claimed in claim 12, wherein the penetration space is set such that at least a partial cross sectional area in an orthogonal direction which is orthogonal to the inserting direction of the analytical tool becomes smaller than a cross sectional area in the orthogonal direction in the analytical tool.

14. The analyzing apparatus having the light shielding means as claimed in claim 13, wherein the penetration space is formed as a taper shape in which the cross sectional area in the orthogonal direction becomes smaller along the inserting direction.

15. The analyzing apparatus having a light shielding means as claimed in claim 11, wherein the contact member has a notch to which the analytical tool is inserted.

16. The analyzing apparatus having the light shielding means as claimed in claim 1, wherein the light measuring mechanism includes a light emitting device, a first light receiving device utilized for monitoring an output of the light emitted from the light emitting device, and a second light receiving device receiving the light transmitting the analytical tool, in the light emitted from the light emitting device.

17. The analyzing apparatus having the light shielding means as claimed in claim 1, wherein the analytical tool is set to a state in which the analytical tool partly protrudes from the analyzing apparatus after starting the insertion of the analytical tool to the installation portion before the light measurement of the analytical tool is finished in the light measuring mechanism.

18. The analyzing apparatus having the light shielding means as claimed in claim 17, wherein the analytical tool is structured such as to suck the sample fed from the portion protruding from the analyzing apparatus into the inner portion thereof.
